(19) Europäisches Patentamt

European Patent Office

Office européen des brevets

(11) Publication number: **0 240 240 B1**

(12) **EUROPEAN PATENT SPECIFICATION**

(45) Date of publication of patent specification: **08.04.92**

(51) Int. Cl.⁵: **A61B 1/26**, F21V 31/00

(21) Application number: **87302607.4**

(22) Date of filing: **25.03.87**

(54) **Submergible laryngoscope battery housing.**

(30) Priority: **01.04.86 US 864913**

(43) Date of publication of application:
**07.10.87 Bulletin 87/41**

(45) Publication of the grant of the patent:
**08.04.92 Bulletin 92/15**

(84) Designated Contracting States:
**AT BE CH DE ES FR GB GR IT LI LU NL SE**

(56) References cited:
**EP-A- 0 169 497**
**DE-A- 1 566 116**
**DE-A- 2 358 042**
**GB-A- 1 205 975**
**US-A- 4 531 178**

(73) Proprietor: **Bauman, Jack**
**16677 San Onofre Drive**
**Pacific Palisades California 90272(US)**

(72) Inventor: **Bauman, Jack**
**16677 San Onofre Drive**
**Pacific Palisades California 90272(US)**

(74) Representative: **Slight, Geoffrey Charles et al**
**Graham Watt & Co. Riverhead**
**Sevenoaks Kent TN13 2BN(GB)**

EP 0 240 240 B1

## Description

This invention generally relates to examining devices such as laryngoscopes and particularly to an improved submersible device of this type.

Laryngoscopes generally comprise a blade and a cooperating handle which are connected together in an L-shaped configuration. The hollow handle normally serves as an enclosure for a power supply such as one or more dry cells which are adapted to energize a light bulb. The light from the bulb passes to the distal end of the blade to illuminate the patient's mouth and larynx during the examination thereof by medical personnel. A surface on the blade is used to press against the tongue and mandible of a patient in a supine position in order to prevent the patient's tongue from obstructing the visual examination of the larynx by medical personnel. A laryngoscope of this kind is disclosed in EP-A-0 169 497

While the instrument is useful for examining the larynx, the primary function of the laryngoscope is to expose the larynx in order to facilitate the insertion of an endotracheal tube. The surface of the laryngoscope blade adjacent the handle is urged against the tongue and mandible to expose the larynx in such procedures and the opposite blade surface is positioned opposing the upper front teeth of the patient.

It becomes desirable to provide a re-usable blade and handle which then must be cleaned thoroughly after use since fluid from the patient's mouth area can contaminate the handle. However, washing of the handle presents the problem of fluid gaining access to the power supply, i.e. dry cells, within the handle hollow, as via one or both ends of the handle. This is a particular problem when the light bulb is carried at the end of the handle to which the blade attaches, as cleaning fluid can leak past the bulb into the handle to cause bulb circuit malfunction.

DE-A-1,566,116 discloses a bronchoscope having a blade, a hollow body means comprising a handle and a cap. The handle is adapted to receive dry cells and has an open end. The cap removably interfits the handle to close its open end and means is provided sealing off between the cap and the handle. Also, means is carried by the cap to pass light from a light source within the body means to a predetermined zone at the cap exterior whereby the light may be transmitted along the blade which is removably attached to the cap. Circuit means including a motion transmitting element to establish an ON condition of the circuit means transmits current from the dry cell means to the light source in use of the device.

DE-A-2,358,042 discloses a flashlamp having a fluid tight handle enclosure for dry cells containing also fluid tight switching means comprising a motion transmitting element movable in response to the manual deflection of a flexible barrier to establish an ON condition of a circuit means for lighting the lamp.

## SUMMARY OF THE INVENTION

Proceeding from the disclosure of DE-A-1,566,116, the present invention provides a laryngoscope having a blade and hollow body means including a handle and a cap, the handle being adapted to receive dry cell means, the handle having an open end, the cap removably interfitting the handle to close said open end, means sealing off between the cap and handle, means carried by the cap to pass light from a light source within said body means to a predetermined zone at the cap exterior, whereby said light may be transmitted along the blade removably attached to the cap, and circuit means including a motion transmitting element to establish an ON condition of the circuit means for transmitting current from said dry cell means to said light source, characterised in that the cap fits the handle telescopically, in that a flexible barrier is carried by the cap to be deflected by the blade upon its attachment to the cap, the barrier (29) sealing off between the cap interior and exterior, in that said motion transmitting element extends proximate said flexible barrier to be deflected thereby, in that said open end of the handle is the sole open end thereof, and in that the handle is formed in one piece.

As will appear, the handle, cap or blade, or combination thereof may consist of light-weight molded plastics material interfitting in such manner as to preserve laryngoscope functions while preventing fluid access to the cap and handle interior during washing. Furthermore, the receptacle may comprise a molded plastics unit having electrically conductive plating thereon, the plating having at least two sections which are not electrically connected, one section being electrically connectible to a dry cell plus terminal, and the other section being electrically connectible to a dry cell negative terminal.

Also, said cap may define two substantially rectangular sub-chambers one receiving said barrier and motion transmitting element, and the other receiving said light source, a light reflector, and a window sealingly engaging an end wall defined by the cap.

One way of carrying out the present invention will not be described in detail by way of example with reference to drawings which show one specific embodiment. In the drawings:-

Fig. 1 is a side elevational view of a laryngoscope at the time of use on a patient, and which embodies features of the invention;

Fig. 2 is a perspective view of the laryngoscope blade and handle end, prior to their assembly;

Fig. 3 is a side elevational view, partially in section, of a laryngoscope, with the blade in ready position;

Fig. 4 is a partial side elevational view, partially in section, of the laryngoscope, with blade in operative position;

Fig. 4a is a cross sectional view taken along lines 4a-4a shown in Fig. 4;

Fig. 5 is a section taken in elevation showing details of the handle portion of the laryngoscope;

Fig. 6 is an end view taken in elevation on lines 6-6 of Fig. 5;

Fig. 7 is a section taken in elevation on lines 7-7 of Fig. 5;

Fig. 8 is an enlarged plan view section taken on lines 8-8 of Fig. 5;

Fig. 9 is an end view taken in elevation on lines 9-9 of Fig. 8;

Fig. 10 is a section on lines 10-10 of Fig. 8;

Fig. 11 is a section on lines 11-11 of Fig. 10;

Fig. 12 is a fragmentary section taken on lines 12-12 of Fig. 8; and

Fig. 13 is a schematic view of circuitry.

## DETAILED DESCRIPTION

The laryngoscope 10, which comprises a handle 11 and blade 12, is utilized to depress the patient's tongue and mandible 13. Frequently, the patient's front teeth 14 are used as a fulcrum for the blade 12, in order to more completely expose the larynx during examination, and to insert an endotracheal tube.

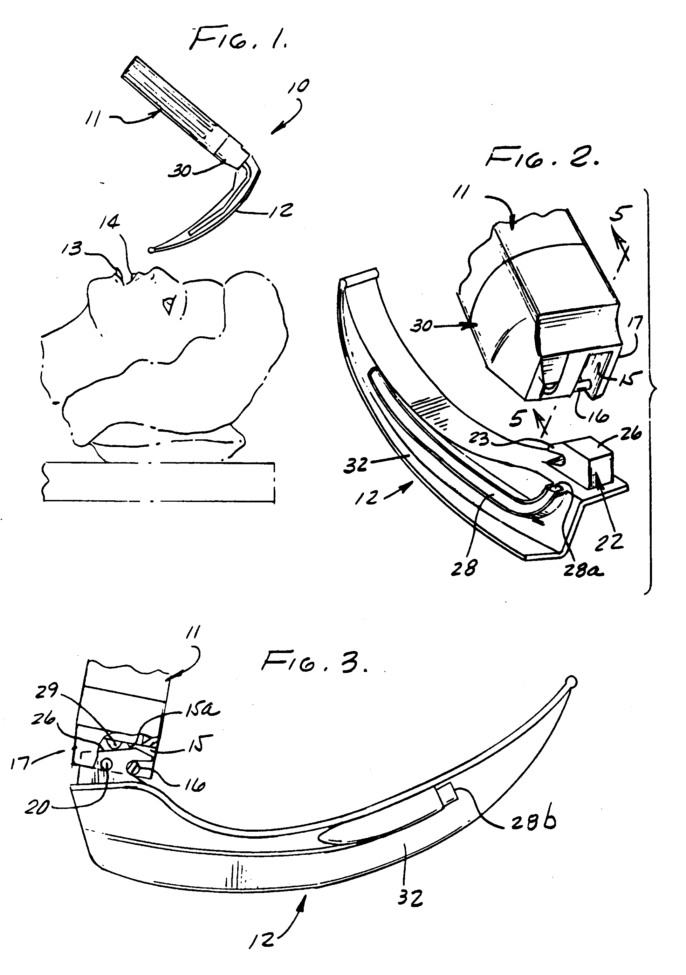

In order to couple the blade 11 to the handle 12, and as shown in Figs. 2 to 4, the upper end of the handle 11 has an open channel 15 provided with a pivot rod 16 extending between flanges 17. The inner side of one flange has a groove or dimple 19 adapted to seat detent 20 projecting at one side surface 18 of a boot shaped appendage on the blade.

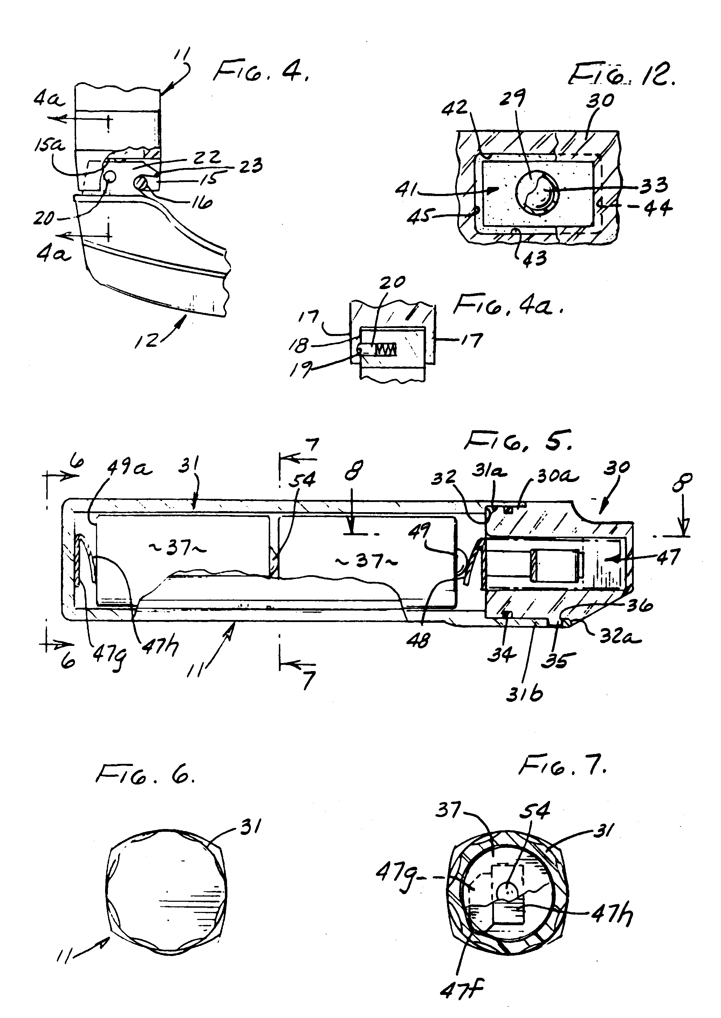

The boot shaped appendage 22 interfits into the open channel 15 and is mounted therein in a pivotal fashion. The front end 23 of the boot shaped appendage 22 is hooked under the pivot rod 16 during the pivotal mounting thereof, in a conventional fashion. To mount the blade onto the handle 11, the appendage 22 of the blade 12 is inserted into the open top channel 15 with a pivotal motion so that the front end 23 rotates under the pivot rod 16 i.e., from Fig. 3 to Fig. 4 condition. The detent 20 moves into engagement with the groove 19 provided in the surface 18, to thereby snap-retain the appendage 22 in firm interfit with the channel and pivot rod 16, as the blade moves from ready position, as shown in Fig. 3, to fixed position seen in Fig. 4.

A light such as an incandescent bulb 25 is provided at the end of the handle proximate the blade to be activated when a light switch 24 is closed by a motion transmitting element 33, as when pushed by bottom surface 26 of appendage or boss 22, via a flexible sealing barrier 29. As shown in Fig. 3, when the blade 12 is initially mounted on the handle 11, the blade 12 is in a ready position on the handle 11 but the bottom surface 26 of the appendage 22 does not activate, i.e. depress, the light switch. Further rotation of the blade 12 causes the detent 20 to engage the groove 19, and to thereby lock the blade 12 in an operating position and simultaneously therewith to cause metallic bottom surface 26 to depress, i.e. activate the light switch 24, which in turn energizes the light source 25.

As best shown in Figs. 2 to 4, light is directed from the light source 25 to ensure the proper illumination of a patient's mouth and larynx when the laryngoscope is being used. The light source or bulb 25 is located near the bottom wall 15a of channel 15 so that, when the blade 12 is rotated into its final operating position, not only is switch 24 engaged, but also the energized bulb directs light into the end 28a of the light pipe or duct 28 carried by the blade and conducting light to the light emitting end 28b directing light forwardly from a channel in blade web 32. Duct 28 may be of optical fiber.

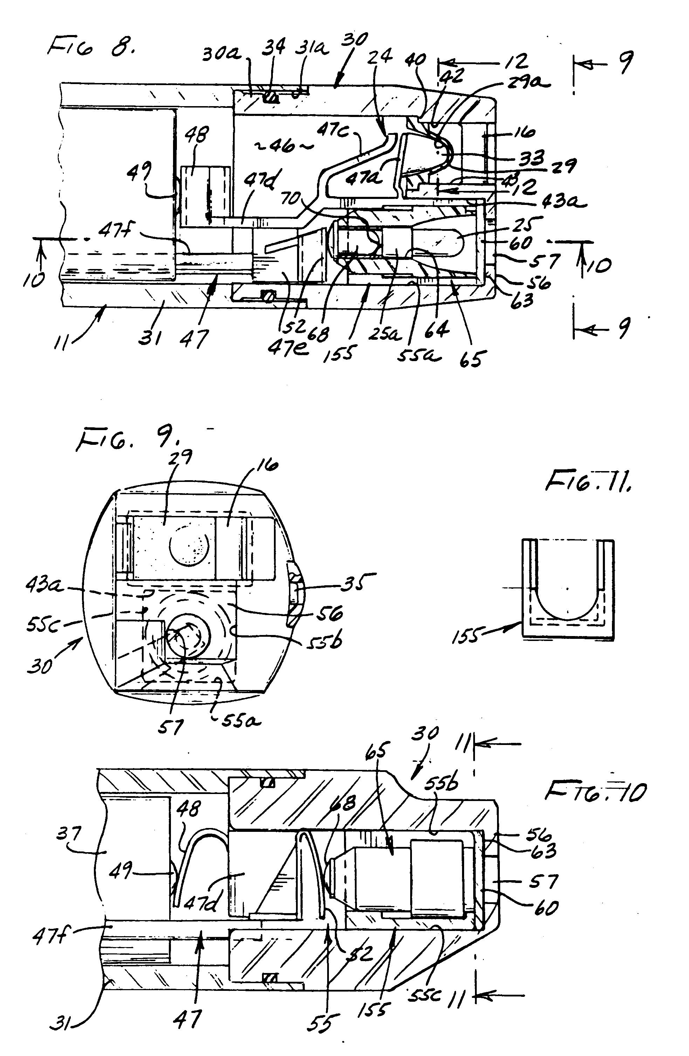

The switch 24 and bulb 25 are both contained in a lightweight plastics cap 30 which fits telescopically into the lightweight plastics handle body 31, as better seen in Figs. 5 and 8. As there shown, the cap 30 has a skirt 30a received into the body bore 31a, to shoulder at 32 and 32a. O-ring seal 34, typically of rubber and carried by skirt 30a, seals off between that skirt and bore 31a. A boss 35 integral with the skirt removably fits or snaps into an opening 36 in the body wall 31b, as shown, to lock the cap in position. Accordingly, the open end of body 31 is closed by the cap. Body 31 receives dry cells 37 that supply electrical energy to the incandescent bulb when switch 24 is closed.

Reference is now made to Fig. 13, showing partially schematically, the integration of circuit means, dry cells, element 33, switch 24, barrier 29, pusher 22 on the blade, and bulb 25. The element 33 may with unusual advantage comprise a nose-shaped part projecting into a corresponding recess 29a formed by the elastomeric barrier 29 which is peripherally retained in position by the cap, as shown at 40 in Fig. 8. In that view, and in Fig. 12 the barrier 29 and part 33 are shown to extend within a sub-chamber 41 formed by cap walls 42 to 45, and the barrier seals off access to the interior 46, via sub-chamber 41. The latter has a substantially rectangular cross section to interfit part 47 to be described.

Returning to Fig. 13, the part 33 may with unusual advantage be integrally molded with an elongated holder or bracket 47 having components 47a to 47h, these being identified further as follows:

--47a - arm supporting part 33 and adapted to bend as blade part 22 pushes against barrier 29 to deflect part 33 leftwardly and close the switch,

--47b - support for arm 47a and fitting cap wall 43a,

--47c - switch terminal to be engaged by part 33, to close the switch,

--47d - projection that supports terminal 48 engaged by battery contact 49,

--47e - support arm that supports 47b, 47c and 47d, and connects them to holder base stretch 47f,

--47f - base stretch that may support batteries 37 to form a sub-assembly insertible into the body 31 and into cap 30,

--47g - connects base stretch 47f to terminal 47h,

--47h - terminal that engages the left battery contact end face 49a.

The said bracket 47 and part 33 thereof may be molded of lightweight strong plastics material and coated with a thin conductive metallic layer, such as silver, except for regions 50 and 51. As a result, when part 33 closes against switch terminal 47c, electrical current flows along the following path: from battery contact 49 through coatings on 47d, 47c 33, and 47b to conductor 52 to bulb 25, then through conductor 53 to coatings on parts 47f, 47g and 47h to the battery contact face 49a. Batteries 37 are of course themselves in end-to-end conductive contact at 54.

Referring now to Figs. 5, 8 and 9, the cap 30 also defines a second sub-chamber 55 which may be rectangular in outline, as defined by walls 43a, 55a, 55b and 55c. End wall 56 of sub-chamber 55 contains an aperture 57 to pass light from bulb 25 to optical fiber light pipe 28, via the end thereof 28a, as previously described. A light passing means in the form of window 60 serves to transmit light from the bulb and reflector 60a toward and onto the end 28a of optical light pipe 28, as also shown in Fig. 13. Window 60 may consist of plastics material and be bonded to the inner surface of end wall 56 at 63, to seal-off the rectangular outline sub-chamber 55 from the exterior, whereby the interior 46 of the handle is kept free from exterior moisture of liquid.

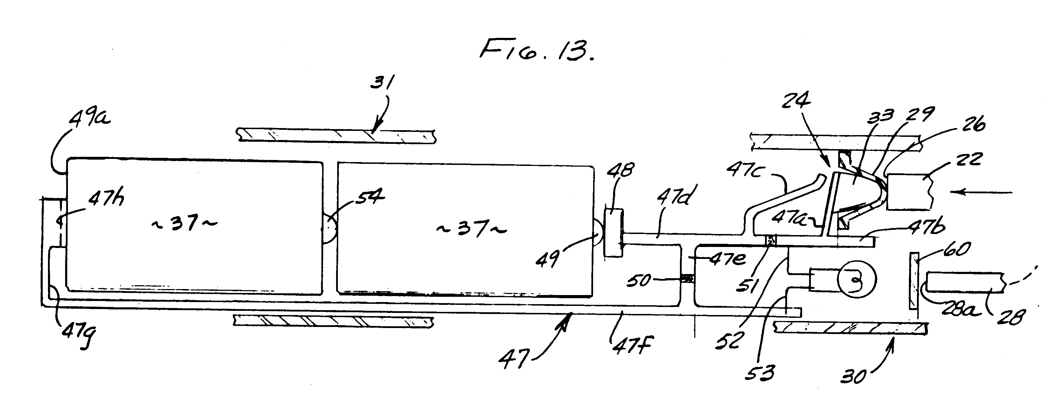

As also shown in Fig. 8, the bulb base 25a is received in bore 64 of a bulb holder 65, which in turn is received in a bracket 155 that fits closely to walls 55a to 55c, and also to part 47b of the receptacle 47. Conductor 53 indicated schematically in Fig. 13 may take the form of conductive holder 155 in Figs. 8 and 9, and conductor 52 in Fig. 13 may take the form in Figs. 8 and 10 of a tab integrally molded with the receptacle 47, and projecting to engage an insert terminal 68 which in turn engages the bulb contact 70.

In the views shown in Figs. 10 and 11 the bracket 155 is shown as U-shaped to receive holder 65. Its exterior is rectangular to fit in the cap rectangular sub-chamber 55.

**Claims**

1. A laryngoscope (10) having a blade (12) and hollow body means including a handle (11) and a cap (30), the handle (11) being adapted to receive dry cell means (37), the handle having an open end, the cap (30) removably interfitting the handle to close said open end, means (34) sealing off between the cap (30) and handle (11), means (60) carried by the cap (30) to pass light from a light source (25) within said body means (11, 30) to a predetermined zone at the cap exterior, whereby said light may be transmitted along the blade (12) removably attached to the cap (30), and circuit means (48, 47, 52, 53, 24) including a motion transmitting element (33) to establish an ON condition of the circuit means (48, 47, 52, 53 24) for transmitting current from said dry cell means (37) to said light source (25), characterised in that the cap (30) fits the handle (11) telescopically, in that a flexible barrier (29) is carried by the cap (30) to be deflected by the blade (12) upon its attachment to the cap (30), the barrier (29) sealing off between the cap interior and exterior, in that said motion transmitting element (33) extends proximate said flexible barrier (29) to be deflected thereby, in that said open end of the handle is the sole open end thereof, and in that the handle is formed in one piece.

2. A laryngoscope as claimed in claim 1, wherein the cap (30) has a first zone (55) to receive a light bulb (25), and a second zone (41) receiving a switch (24) which, when closed, completes a circuit between the power supply (37) and the light bulb (25), a window (26) carried by the cap (30) and closing a first opening (57) in the cap to pass light from the light bulb (25), the first and second zones (55, 41) being located in side-by-side relation, only said flexible barrier separating said second zone from the exterior of the cap.

3. A laryngoscope as claimed in claim 2, wherein said cap (30) has a wall (15a) defining a second opening (40) across which said flexible barrier (29) extends, to block fluid access from the cap exterior via said second opening (40) to the interior of the cap.

4. A laryngoscope as claimed in claim 3, wherein each of said openings (57, 40) is substantially rectangular in cross section.

5. A laryngoscope as claimed in any preceding claim, wherein said circuit means includes a battery receptacle (47) received endwise into said hollow handle (11) and into said cap (30) to interfit same and said motion transmitting element (33) is integral with said receptacle and positioned to extend proximate said flexible barrier to be deflected thereby, and switch terminals (47a, 47c) are carried by said receptacle (47) to be closed together in response to said deflection of the motion transmitting element (33).

6. A laryngoscope as claimed in any preceding claim, wherein said cap (30) defines two substantially rectangular sub-chambers (41, 55) one receiving said barrier (29) and motion transmitting element (33), and the other receiving said light source (25), a light reflector (60a), and a window (60) sealingly engaging an end wall (15a) defined by the cap (30).

7. A laryngoscope as claimed in any preceding claim, wherein each of said handle (11) cap (30) and blade (12) consist of molded plastics material.

**Revendications**

1. Laryngoscope (10) comportant une lame (12) et un moyen de corps creux comportant une poignée (11) et un bouchon (30), la poignée (11) étant prévue pour recevoir un moyen de pile sèche (37), la poignée ayant une extrémité ouverte, le bouchon (30) s'adaptant de manière amovible à la poignée pour fermer ladite extrémité ouverte, un moyen (34) fermant hermétiquement l'espace entre le bouchon (30) et la poignée (11), un moyen (60) supporté par le bouchon (30) pour passer la lumière depuis une source lumineuse (25) à l'intérieur dudit moyen de corps (11, 30) à une zone prédéterminée à l'extérieur du bouchon, si bien que ladite lumière peut être transmise le long de la lame (12) fixée de manière amovible au bouchon (30), et un moyen de circuit (48, 47, 52, 53, 24) comportant un élément de transmission de mouvement (33) pour établir un état en service du moyen de circuit (48, 47, 52, 53, 24) pour transmettre le courant depuis ledit moyen de pile sèche (37) vers ladite source lumineuse (25), caractérisé en ce que le bouchon (30) adapte la poignée (11) de manière télescopique, en ce qu'une barrière flexible (29) est supportée par le bouchon (30) pour être déviée par la lame 12 sur sa fixation au bouchon (30), la barrière (29) fermant l'espace entre l'intérieur du bouchon et l'extérieur, en ce que ledit élément de transmission de mouvement (33) s'étend près de ladite barrière flexible (29) pour être dévié par celle-ci, en ce que ladite extrémité ouverte de la poignée est la seule extrémité ouverte de celle-ci, et en ce que la poignée est formée en un seul tenant.

2. Laryngoscope selon la revendication 1, dans lequel le bouchon (30) comporte une première zone (55) pour recevoir une ampoule lumineuse (25) et une seconde zone (41) pour recevoir un interrupteur (24) qui, lorsque fermé, ferme un circuit entre l'alimentation (37) et l'ampoule lumineuse (25), une fenêtre (26) supportée par le bouchon (30) et fermant une première ouverture (57) dans le bouchon pour laisser passer la lumière depuis l'ampoule lumineuse (25), les première et seconde zones (55, 41) étant situées en relation côte à côte, seulement ladite barrière flexible séparant la seconde zone de l'extérieur du bouchon.

3. Laryngoscope selon la revendication 2, dans lequel ledit bouchon (30) comporte une paroi (15a) définissant une seconde ouverture (40) à travers laquelle ladite barrière flexible (29) s'étend, pour bloquer l'accès d'un fluide depuis l'extérieur du bouchon par l'intermédiaire de la seconde ouverture (40) à l'intérieur du bouchon.

4. Laryngoscope selon la revendication 3, dans lequel chacune desdites ouvertures (57, 40) est pratiquement rectangulaire en section transversale.

5. Laryngoscope selon l'une quelconque des revendications précédentes, dans lequel ledit moyen de circuit comporte un logement de pile (47) reçu dans le sens de la longueur dans ladite poignée creuse (11) et dans ledit bouchon (30) pour s'entradapter et ledit élément de transmission de mouvement (33) est solidaire avec ledit logement positionné pour s'étendre proche de ladite barrière flexible pour être dévié par celle-ci, et les bornes de l'interrupteur (47a, 47b) sont supportées par ledit logement (47) pour être fermées en même temps en réponse à ladite déviation de l'élément de transmission de mouvement (33).

6. Laryngoscope selon l'une quelconque des revendications précédentes, dans lequel ledit bouchon (30) définit deux sous-chambres pratiquement rectangulaires (41, 51), une recevant ladite barrière (29) et l'élément de transmission de mouvement (33), et l'autre recevant ladite source lumineuse (25), un réflecteur de lumière (60a) et une fenêtre engageant de manière étanche (60) une paroi d'extrémité (15a) définie par le bouchon (30).

7. Laryngoscope selon l'une quelconque des revendications précédentes, dans lequel chacun desdites poignées (11) du bouchon (30) et de la lame (12) sont constituées de matière plastique moulée.

## Patentansprüche

1. Laryngoskop (10) mit einem Blatt (12) und einem einen Handgriff (11) und eine Kappe (30) umfassenden Hohlkörper, wobei der Handgriff (11) zur Aufnahme einer Trockenzelleneinrichtung (37) geeignet ist, der Handgriff ein offenes Ende besitzt und die Kappe (30) abnehmbar am Handgriff zum Schließen des offenen Endes angesetzt ist, sowie mit einer Abdichtung (34) zwischen der Kappe (30) und dem Handgriff (11), einer von der Kappe (30) getragenen Einrichtung (60) zum Durchleiten von Licht von einer Lichtquelle (25) innerhalb des Hohlkörpers (11,30) zu einer vorbestimmten Zone am Kappenäußeren, wodurch das Licht entlang dem abnehmbar an der Kappe (30) angebrachten Blatt (12) übertragen werden kann, und einem Stromkreis (48, 47, 52, 53, 24) mit einem Bewegungsübertragungselement (33) zur Errichtung eines AN-Zustands des Stromkreises (48, 47, 52, 53, 24) zur Übertragung von Strom von der Trockenzelleneinrichtung (37) zu der Lichtquelle (25), dadurch gekennzeichnet, daß die Kappe (30) teleskopartig an den Handgriff (11) angesetzt ist, daß eine flexible Absperrung (29) von der Kappe (30) gehalten ist, um von dem Blatt (12) bei seiner Anbringung an der Kappe (30) ausgelenkt zu werden, wobei die Absperrung (29) zwischen dem Kappeninneren und -äußeren abdichtet, daß sich das Bewegungsübertragungselement (33) nahe an die flexible Absperrung (29) heranerstreckt, um von dieser ausgelenkt zu werden, daß das offene Ende des Handgriffs dessen einziges offenes Ende ist und daß der Handgriff in einem Stück gebildet ist.

2. Laryngoskop nach Anspruch 1, bei dem die Kappe (30) eine erste Zone (55) zur Aufnahme einer Lichtröhre (25) und eine zweite Zone (41) besitzt, die einen Schalter (24) aufnimmt, der im geschlossenen Zustand einen Stromkreis zwischen der Stromversorgung (37) und der Lichtröhre (25) schließt, ein Fenster (26) von der Kappe (30) getragen ist und eine erste Öffnung (57) in der Kappe zum Hindurchleiten von Licht von der Lichtröhre (26) schließt, die erste und die zweite Zone (55, 41) Seite an Seite angeordnet sind und nur die flexible Absperrung die zweite Zone von der Außenseite der Kappe trennt.

3. Laryngoskop nach Anspruch 2, bei dem die Kappe (30) eine Wand (15a) besitzt, die eine zweite Öffnung (40) begrenzt, über die sich die flexible Absperrung (29) erstreckt, um einen Flüssigkeitszutritt von der Außenseite der Kappe über die zweite Öffnung (40) zur Innenseite der Kappe zu verhindern.

4. Laryngoskop nach Anspruch 3, bei dem jede der Öffnungen (57,40) im wesentlichen einen rechteckigen Querschnitt besitzt.

5. Laryngoskop nach einem der vorhergehenden Ansprüche, bei dem der Stromkreis eine Batterieaufnahme (47) aufweist, die der Länge nach in dem hohlen Handgriff (11) und der Kappe (30) aufgenommen ist, um diese zusammenzufügen, und das Bewegungsübertragungselement (33) einstückig mit der Aufnahme ausgebildet und so angeordnet ist, daß es sich nahe an die flexible Absperrung erstreckt, um von dieser ausgelenkt zu werden, und Schaltterminals (47a, 47c) von der Aufnahme (47) getragen sind, um als Folge der Auslenkung des Bewegungsübertragungselements (33) geschlossen zu werden.

6. Laryngoskop nach einem der vorhergehenden Ansprüche, bei dem die Kappe (30) zwei im wesentlichen rechteckige Unterkammern (41,55) bildet, von denen die eine die Absperrung (29) und das Bewegungsübertragungselement (33) aufnimmt und die andere die Lichtquelle (25), einen Lichtreflektor (60a) und ein Fenster (60) aufnimmt, das in Dichtungseingriff mit einer von der Kappe (30) gebildeten Endwand (15a) steht.

7. Laryngoskop nach einem der vorhergehenden Ansprüche, bei dem der Handgriff (11), die Kappe (30) und das Blatt (12) jeweils aus geformtem Kunststoffmaterial bestehen.

FIG. 1.

FIG. 2.

FIG. 3.

8

FIG. 4.

FIG. 12.

FIG. 4a.

FIG. 5.

FIG. 6.

FIG. 7.

FIG 8.

FIG. 9.

FIG. 11.

FIG. 10

Fig. 13.